# EUROPEAN PATENT SPECIFICATION

(11) **EP 4 198 616 B1**
(45) Date of publication and mention of the grant of the patent: **07.10.2026**
(21) Application number: 21306823.2
(22) Date of filing: 17.12.2021
(51) Int. Cl.: G02C 7/02

(54) **A METHOD AND DEVICE FOR DETERMINING AN OPTIMIZED VISUAL EQUIPMENT**
VERFAHREN UND VORRICHTUNG ZUR BESTIMMUNG EINER OPTIMIERTEN VISUELLEN AUSRÜSTUNG
PROCÉDÉ ET DISPOSITIF PERMETTANT DE DÉTERMINER UN ÉQUIPEMENT VISUEL OPTIMISÉ

(43) Date of publication of application: 21.06.2023
(73) Proprietor: Essilor International, 94220 Charenton-Le-Pont (FR)
(72) Inventor: TATUR, Guillaume, 77144 MONTEVRAIN (FR); CALIXTE, Laurent, 75012 PARIS (FR); FRICKER, Sébastien, 94000 CRETEIL (FR)
(74) Representative: Ipsilon

(56) References cited:
- AU-A1- 2020 246 957
- AU-A1- 2020 247 193
- US-A1- 2019 113 770

## Description

### FIELD OF THE INVENTION

The present invention relates to a method and device for determining an optimized visual equipment.

### BACKGROUND OF THE INVENTION

In order to design an ophthalmic lens, a lens designer generally needs to know at what distances or proximities the objects looked at by the wearer are located and what gaze directions are used by the wearer to look at a given object.

In the state of the art, this information is gathered in an ergorama, which is a function associating to each gaze direction the usual distance or proximity of an object point. Based on the ergorama, the designer can thus determine for each gaze direction the proximity of the object.

Using the proximity of the object, ray-tracing can be performed to determine the power, astigmatism and other optical characteristics of the lens.

The lens power can be compared to the object proximity and to the wearer's prescription to determine if the lens is satisfactory. Similarly, the lens astigmatism can be compared to the wearer's prescription to determine if the lens is satisfactory.

An ideal power distribution linked to an ergorama is obtained, but with no certainty that this ideal power distribution corresponds to a realistic lens design. Furthermore, an ergorama associates an object point to each direction, but does not make it possible to manage cases where a single direction could be used to look at several object points, for example object points at different distances.

Ideally, one would like to decouple the environment information, i.e. where the objects are located with respect to the wearer, from the behavior information, i.e. how the wearer will be looking at these objects through the lens, as well as from the lens characteristics, i.e. how the lens deviates light. Using an ergorama implies coupling these three aspects. Therefore, optimizing lenses using ergoramas provides sub-optimal lens designs.

Also, it would be useful to adapt a lens to specific environments, for example an office, a car driver's seat, etc. In the state of the art, this requires to redefine ergoramas in each case.

Further, it would be useful to adapt a lens to a specific wearer's visual behavior, for example taking account of the fact that the wearer is a kid.

In addition, ergoramas imply prismatic deviation values. Prismatic deviations depend on the lens power and design. So, theoretically, each lens should have a different ergorama. This is complicated to implement in practice.

More generally, although state of the art lens computation tends to fit the wearer's needs, in various daily life situations, wearers may experience discomfort and are posturally constrained during the visual exploration of their usual environments. These drawbacks are related to the limited usage of realistic tridimensional environments in the object space, as well as to the lack of postural effort information to drive the lens computation and optimization process. From an optimization point of view, there is a challenge in using simultaneously values related to optical performance and wearer centered criteria.

Documents WO 2020/193436 A1 and WO 2020/193370 A1 disclose devices and methods for evaluating a performance of a visual equipment for a visual task.

Document US 2019/0113770 A1 discloses a method for determining an ophthalmic lens adapted to a locomotion parameter.

For all the above reasons, there is a need to be able to optimize a lens without the use of an ergorama, in order to manage both quality of vision and efforts in visual behavior.

### SUMMARY OF THE INVENTION

An object of the present disclosure is to overcome the above-mentioned drawbacks of the prior art.

To that end, the invention relates to a method according to claim 1.

Thus, the method according to the disclosure makes it possible to compute an ophthalmic lens taking into account a tridimensional environment and a virtual wearer including the wearer's postural efforts and visual needs, without making use of an ergorama.

The method according to the present disclosure may be used by a lens designer to create a lens for an average wearer or a population of wearers. It may also be used by an Eye Care Professional (ECP) to optimize or adapt a lens to an individual wearer's specific environment or characteristics. In that case, the method according to the present disclosure may be an application used by the ECP.

To the same end as mentioned above, the invention also provides a device according to claim 11.

To the same end as mentioned above, the invention also provides a computer program product according to claim 12.

To the same end as mentioned above, the invention further provides a non-transitory information storage medium according to claim 13.

As advantages of the device, computer program product and information storage medium are similar to those of the method, they are not repeated here.

The computer program product is advantageously configured for executing the method in any of its execution modes.

### BRIEF DESCRIPTION OF THE DRAWINGS

For a more complete understanding of the description provided herein and the advantages thereof, reference is now made to the brief descriptions below, taken in connection with the accompanying drawings and detailed description, wherein like reference numerals represent like parts.
FIG. 1 is a flow diagram showing steps of a method according to the present disclosure, in a particular embodiment.
FIG. 2 is a schematic view of an example of a model of a wearer obtained in a method according to the present disclosure, in a particular embodiment.
FIG. 3 is a schematic view of an example of a model of an environment obtained in a method according to the present disclosure, in a particular embodiment.
FIG. 4 is a pair of graphs illustrating a non-limiting example of multi-objective optimization used in a particular embodiment of a method according to the present disclosure.

### DETAILED DESCRIPTION OF EMBODIMENTS

In the description which follows, the drawing figures are not necessarily to scale and certain features may be shown in generalized or schematic form in the interest of clarity and conciseness or for informational purposes. In addition, although making and using various embodiments are discussed in detail below, it should be appreciated that as described herein are provided many inventive concepts that may embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features relative to a device can be transposed, individually or in combination, to a process, in accordance with the appended claims.

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof such as "includes" and "including") are open-ended linking verbs. They are used to specify the presence of stated features, integers, steps or components or groups thereof, but do not preclude the presence or addition of one or more other features, integers, steps or components or groups thereof. As a result, a method, or a step in a method, that "comprises", "has", "contains", or "includes" one or more steps or elements possesses those one or more steps or elements, but is not limited to possessing only those one or more steps or elements.

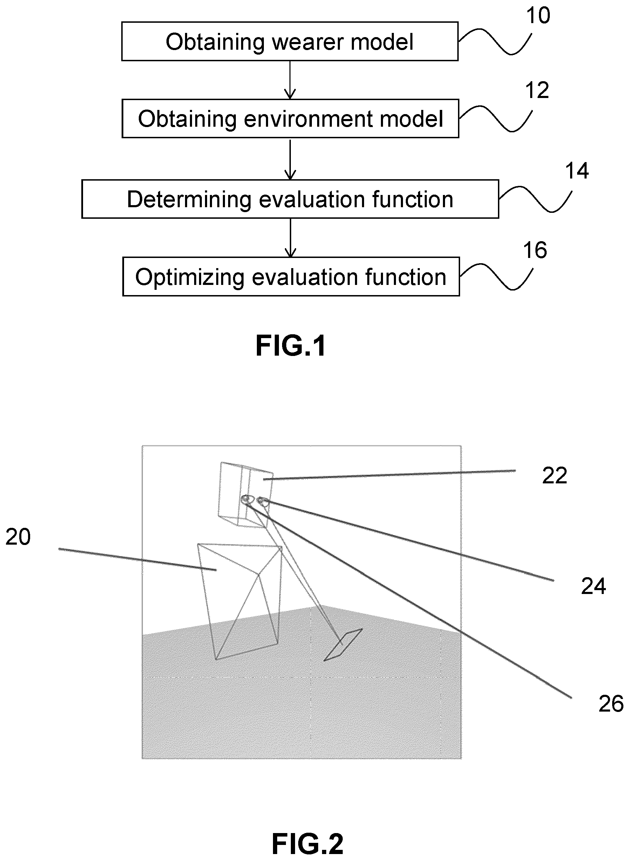

Figure 1 shows steps of a method according to the present disclosure for determining at least one optimized visual equipment to be worn by a human wearer, in a particular embodiment.

The visual equipment may be an ophthalmic lens or pair of ophthalmic lenses, or a solar lens or pair of solar lenses, or an ophthalmic solar lens or pair of ophthalmic solar lenses. It may be in the form of glasses or contact lenses.

The characteristics of the visual equipment may be selected among real existing equipments, or constructed as desired, as a virtual visual equipment.

The human wearer may be either an existing person, or be representative of a predetermined group of users or deemed to be an average user.

The human wearer may be chosen from a database of multiple wearers, in which the wearers are organized in clusters according to a particular characteristic, such as age, gender, ethnicity, activity, refraction, etc.

The expression "the wearer" means that the person is wearing the visual equipment.

A first step 10 of the method comprises obtaining a wearer model, which is a virtual model of the human wearer.

A following step 12 comprises obtaining a model of at least one environment for which the at least one optimized visual equipment is to be determined. The environment comprises tridimensional positions of objects to be viewed by the wearer model.

The wearer may be modeled as a set of rigid body parts capable of moving with respect to each other.

As shown in Figure 2, in a particular embodiment, the model of the wearer comprises a trunk 20, a head 22 rotationally movable with respect to the trunk 20 and at least one eye 24 rotationally movable with respect to the head 22. The at least one eye 24 is a means for the head 22 for looking at a given point without being bound to the rotation of the head 22. At least one ophthalmic and/or solar lens 26 pertaining to the visual equipment cooperates with the eye in front of which it is positioned.

The lens or lenses 26 may be defined by their geometry i.e. shape, material or refractive index, and position with respect to the eye(s) and/or head of the wearer.

The trunk 20 may be considered as static in the main reference frame of the environment model for the duration of a visual task.

As a variant, the model of the wearer may comprise a predetermined motion of the trunk 20. For example, the motion of the trunk 20 may be a typical trunk motion that has been previously recorded, such as the motion of the trunk during a given activity: walking, running, climbing stairs, etc. In addition, optionally, an effort of the wearer to move the trunk with respect to the waist may be taken into account in the wearer model.

Optionally, the model of the wearer may further comprise other parameters relating to the wearer, such as data relating to the size of the wearer and/or the manner in which the wearer is moving and/or a particular type of visual deficiency of the wearer, e.g. myopia or hypermetropia, usage and lifestyle specifications, i.e. at least one personalized parameter related to the human wearer.

Regarding the motion of the head 22, several models of head motions are known, for example from the article by M. Kunin et al. entitled "Rotation Axes of the Head During Positioning, Head Shaking, and Locomotion", in J. Neurophysiol. 98, pages 3095-3108, 2007.

The reference frame of the trunk may be defined as follows. The origin of the trunk reference frame is located at the midpoint between the left shoulder point and the right shoulder point. The X-axis of the trunk reference frame passes through the shoulder points and points toward the right side of the wearer in a direction corresponding to the horizontal direction. The Z-axis points toward the back of the wearer in the horizontal direction and is perpendicular to the line joining the two shoulder points. The Y-axis points upwards, in a direction corresponding to the vertical direction and is orthogonal to the X-axis and to the Z-axis.

The reference frame of the head may be defined as follows. The origin of the head reference frame is located at the midpoint between the two tragions. The X-axis of the head reference frame passes through the two tragions and points toward the right side of the wearer in a direction corresponding to the horizontal direction. The Z-axis of the head reference frame point toward the back of the wearer in the horizontal direction and is perpendicular to the line joining the two tragions. The Y-axis points upwards, in a direction corresponding to the vertical direction and is orthogonal to the X-axis and to the Z-axis.

For example, the head motion may be described by three angles theta, phi and rho, corresponding respectively to head pitch, yaw and roll. For example, the transformation matrix from the reference frame of the trunk to the reference frame of the head may be the combination of a rotation about an X-axis of an angle equal to k x theta, a translation about an Y-axis, orthogonal to the X-axis, of a distance equal to the trunk to head distance, a rotation about a Z-axis, orthogonal to the X-axis and to the Y-axis, of an angle equal to rho, a rotation about the X-axis of an angle equal to (1-k) x theta and a rotation about the Y-axis of an angle equal to phi, where k = 0.22.

The eye motions may for example be described by two angles corresponding to eye gaze directions: lowering and azimuth. Optionally, the torsion of the eye may also be taken into account. For example, the eye motions may be described according to Listing's law.

The model of the environment comprises at least one object defined by at least one geometric parameter and by a position of the object in the environment, the objects being associated with gaze fixation points, visual requirement parameters and at least one criterion. The environment model may be represented as a tridimensional cloud of points. Optimal viewing of the objects will be evaluated by computing at least one criterion, such as head comfort area, postural effort, monocular or binocular visual acuity, relative to optimal values for each of the fixation points. The head comfort area or head freedom area of an ophthalmic lens corresponds to the head positions of the wearer that provide optical performance greater than or equal to a specific threshold value when the wearer is wearing the ophthalmic lens in given wearing conditions upon given fitting parameters and is looking at a specific point in a scene through the ophthalmic lens. The optical performance can be based on power error, unwanted astigmatism, and according to the claimed subject-matter on visual acuity or visual acuity loss and can also include a threshold value linked with postural efforts.

Additionally, wearer lifestyle and usage may specify a criterion relevance weight and an object usage coefficient to be used in an evaluation function, as described in more detail below.

Other features of the environment may be included in the model, including color, texture, contrast, light sources, etc. The model of the environment may also take into account at least one personalized parameter related to a real environment in which the human wearer intends to use the visual equipment.

In a particular embodiment, the method further comprises defining one or more visual tasks, by associating with each other a plurality of fixation points to be looked at by the wearer model in the model of the at least one environment. Thus, the plurality of fixation points forms a sequence of points that is the visual task model. Optionally, the time instant at which each point is looked at may be included in the model of the visual task.

Additional parameters may be included in the model of the visual task, such as the minimum visual acuity or the maximum visual acuity loss with which a given fixation point must be seen, or the time interval between one fixation point and the next one.

Moreover, additional visual task parameters may be taken into account in the model of the visual task, such as visual fatigue, attention, peripheral vision, fusion, etc.

In other words, the model of the visual task may be a list of fixation points also referred to as fixations, where each fixation may be defined by its position on a given object of the scene, either in a predetermined reference frame of the object, or in a predetermined "main" reference frame in the model of the environment, with optionally the minimum visual acuity or maximum visual acuity loss for the fixation, also optionally, the duration of the fixation and also optionally, any other visual task parameters.

A first non-limiting example of a visual task is as follows. A computer screen is placed in front of the wearer, at a distance of 70 cm. A grid of points is defined which covers the screen and which represents the position of the saccades when a wearer is reading a text on the computer screen. The visual task consists in looking at each point of the grid, sequentially, from top left to bottom right, with an acuity loss lower than or equal to 0.1 logMAR (with reference to the Logarithm of the Minimum Angle of Resolution) at each point.

A second non-limiting example of a visual task may be as follows. A television screen is placed in front of the wearer, at a distance of 3 m. A random set of points is defined on the screen, which represent the positions of the fixation points when a wearer is watching a movie. The visual task consists in looking at each point of the set of points, sequentially, in a random order, with an acuity loss lower than or equal to 0.2 logMAR.

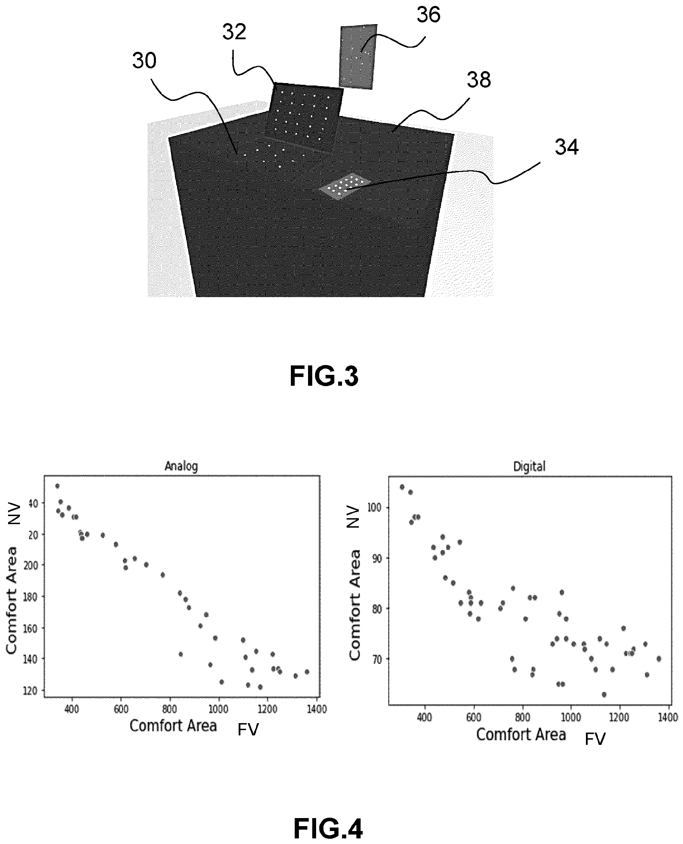

Figure 3 gives a non-limiting example of a model of an environment including a laptop keyboard 30, a laptop screen 32, a smartphone 34 and a person 36 standing in front of the wearer. The smartphone 34 and the laptop, comprising the laptop keyboard 30 and the laptop screen 32, are located on a desk 38. The points to be looked at in the environment are defined and shown in Figure 3 on the laptop keyboard 30, the laptop screen 32, the smartphone 34 and the person 36. In this example, a visual task may consist in looking sequentially at each point defined in the environment in a predefined order.

More generally, the model of the environment may comprise a main reference frame. The model of the environment may further comprise one or more objects, each object having its own reference frame. The shape of an object may comprise geometric forms defined in the reference frame of the object, such as points, lines, rectangles, spheres, parallelepipeds, triangular meshes and/or quad meshes. The position of an object in the environment may be defined with respect to the main reference frame.

For example, the position of an object with respect to the main reference frame may be described by using a rotation matrix R and a translation matrix T. For example, the rotation matrix R is a 3x3 matrix and the translation matrix T is a 3x1 vector.

The coordinates of a given point in the reference frame of an object are given by a triplet Po and the coordinates of that point in the main reference frame are given by a triplet Pm. The transformation from the main reference frame to the reference frame of the object is given by (R, T) such that Po = R x Pm + T.

Each object may thus be defined by its geometry and the transformation (R, T).

As shown in Figure 1, after step 10 of obtaining the wearer model and step 12 of obtaining the environment model, a step 14 of the method according to the present disclosure comprises determining at least one evaluation function related to the visual equipment.

The evaluation function is a function of the at least optical performance of the visual equipment and postural performance of the wearer model in the environment model.

In a particular embodiment, the evaluation function comprises at least one weighting coefficient related to the human wearer's lifestyle, which relates to at least one type of activity in the course of which the human wearer intends to use the visual equipment. The at least one weighting coefficient may be modified for taking into account preferences or usages of the human wearer.

In a particular embodiment where at least one visual task is defined by associating with each other a plurality of fixation points to be looked at by the wearer model in the environment model, the at least one evaluation function measures the optical performance of the visual equipment and the postural performance of the wearer model when the wearer model is carrying out the at least one visual task.

A non-limiting example of wearer usage and lifestyle may comprise using a smartphone for videos, a laptop for office work and wearing single vision lenses as a previous visual equipment.

In that example, the following criteria and object usage coefficients may be chosen:
For the smartphone, located at 35 cm from the wearer:
Criterion 1: acuity loss, target value: 0.15 logMAR, weight: 1
Criterion 2: postural effort, target value: postural effort for single vision equipment, weight: 2
Smartphone coefficient: 0.5
For the laptop, located at 70 cm from the wearer:
   Criterion 1: head comfort area, target value: 350°, weight: 2
   Criterion 2: postural effort, target value: postural effort for single vision equipment, weight: 2
   Laptop coefficient: 2
   For far vision:
      Criterion 1: acuity loss, target value: 0.1 logMAR, weight: 1
      Criterion 2: postural effort, target value: postural effort for single vision equipment, weight: 1
      Criterion 3: optical/retinal flow, target value: optical/retinal flow for single vision equipment, weight: 2
      Far vision coefficient: 1

A description of the optical/retinal flow can be found in document US 2019/113770 A1, at page 3.

The optical performance comprises visual acuity or visual acuity loss.

The postural performance comprises at least one of a head comfort area, a postural effort, a gaze effort and a combined postural and gaze effort.

The punctual physiological effort may for example comprise a head posture effort, which is the physiological effort made by the model of the wearer for having such a head posture.

A head posture effort function may compute the effort made by the wearer to maintain a position of the head. Typically, the posture effort is minimum for a head lowering, head azimuth and head torsion angles of zero degree. The punctual physiological effort may for example comprise, instead of the head posture effort or in addition to the head posture effort, a gaze effort of at least one eye, which is the physiological effort made by the model of the wearer for having such a gaze direction.

A gaze effort function may compute, for at least one eye, the effort made by the wearer to maintain a position as a function of the gaze direction. Typically, the gaze effort is minimum for a gaze lowering angle of about 15 degrees and a gaze azimuth angle of zero degree.

A visual acuity model may compute, for a given object point and wearer head posture, the visual acuity with which the point is seen by the wearer. This model may take into account the aberrations of the lens in the gaze direction of the wearer. It may also take into account an accommodation value of the wearer or the maximum visual acuity of the wearer. A visual acuity model such as the one described in document WO 2017/064065 A1 may be used.

Each of the head posture effort function, gaze effort function and visual acuity model may be any of an average model, a model built on the basis of measurements made for a given wearer, or a model for a segment or cluster of wearers having for example a common particular characteristic such as gender, age, ethnicity, activity, refraction, etc.

By way of non-limiting example, the gaze effort of one eye may be defined as follows: alpha is the eye's gaze lowering angle in degrees, beta is the eye's gaze azimuth angle in degrees and GazeEffort is the eye's gaze effort in arbitrary units. $a = alpha / 100$ $b = beta / 100$ 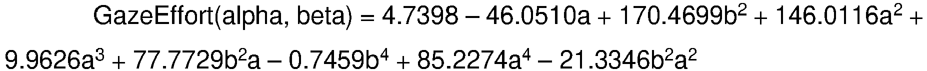 GazeEffort(alpha, beta) = 4.7398 - 46.0510a + 170.4699b2 + 146.0116a2 + 9.9626a3 + 77.7729b2a - 0.7459b4 + 85.2274a4 - 21.3346b2a2

By way of non-limiting example, the head posture effort may be defined as follows. Referring to the head rotation angles theta, phi and rho, corresponding respectively to head pitch, yaw and roll, theta is the rotation angle of the head about the X-axis in degrees, phi is the rotation angle of the head about the Y-axis in degrees, rho is the rotation angle about the Z-axis in degrees, all rotations being defined with respect to the trunk, and HeadPostureEffort is the head posture effort in arbitrary units. $HeadPostureEffort \left(theta, phi, rho\right) = ex + ey + ez ,$ where: $ex = {\left(theta/80\right)}^{2}$ $ey = {\left(phi/80\right)}^{2}$ $ez = {\left(rho/45\right)}^{2}$
1) For a given head posture defined by the head rotation angles (theta, phi, rho), the gaze directions of the left eye, GazeDirectionLeftEye(theta, phi, rho) and/or of the right eye GazeDirectionRightEye(theta, phi, rho) for fixation of the given object point through the lenses may be computed, for example by ray tracing, taking into account deviations by the lenses:
   alpha_left, beta_left = GazeDirectionLeftEye(theta, phi, rho)
   alpha_right, beta_right = GazeDirectionRightEye(theta, phi, rho)
   where alpha_left is the gaze lowering angle of the left eye, beta_left is the gaze azimuth angle of the left eye, alpha_right is the gaze lowering angle of the right eye and beta_right is the gaze azimuth angle of the right eye.
2) From the gaze directions of the left and right eyes, the gaze effort for the left eye, gaze_effort_left and the gaze effort for the right eye, gaze_effort_right, may be evaluated:
   gaze_effort_left = GazeEffort(alpha_left, beta_left)
   gaze_effort_right = GazeEffort(alpha_right, beta_right)
3) From the head rotation angles theta, phi and rho, the head posture effort, head_posture_effort, may be evaluated:
   head_posture_effort = HeadPostureEffort(theta, phi, rho)
4) Optionally, if the object point is associated with a target visual acuity value, the visual acuity with which the point is seen, acuity_value, may be evaluated:
   acuity_value = AcuityModel(alpha_left, beta_left, alpha_right, beta_right)
5) If the visual acuity, acuity_value, with which the point is seen is lower than the target visual acuity, acuity_target, a visual acuity penalty, acuity_penalty, may be computed:
   acuity_penalty = max(acuity_target - acuity_value, 0)
   where max(acuity_target - acuity_value, 0) is the highest value between 0 and the difference between the visual acuity target value and the actual visual acuity value.

The parameters defining the model of the wearer may be average parameters.

As a variant, they may be segmented parameters, i.e. parameters of a predetermined category or cluster of wearers. As mentioned previously, non-limiting examples of such categories are gender (man or woman), age (child, or young adult, or adult, or senior), ethnicity, activity, refraction, etc.

As another variant, the parameters defining the model of the wearer may be personalized i.e. they may be the parameters of an individual wearer.

The parameters that may be segmented or personalized include: the gaze effort model as a function of gaze direction and/or the head posture effort model as a function of the head posture and/or the visual acuity model as a function of lens aberrations and/or the prescription for at least one eye and/or the position of at least one eye in the head and/or the ranges of motion of at least one eye and/or the ranges of motion of the head.

In a particular embodiment, the method according to the present disclosure further comprises defining target optimal values related to the optical performance and the postural performance, in which case the at least one evaluation function is a sum of weighted normalized differences between evaluated values of the optical and postural performances and the target optimal values. Each weight may be defined as the product of a criterion relevance weight and an object usage coefficient. Additionally, the target optimal values may be specific to each criterion, e.g. 0.10 logMAR for an acuity loss criterion, or 300° for a head comfort area criterion.

A final step 16 of the method according to the invention comprises optimizing the at least one evaluation function, so as to determine the at least one optimized visual equipment. The visual equipment is parameterized and the parameters that define the visual equipment are the variables to optimize. On the basis of the minimization of the evaluation function, an optimal visual equipment can be determined.

In a particular embodiment, step 14 of determining the at least one evaluation function comprises determining a set of evaluation functions. In such an embodiment, step 16 of optimizing the at least one evaluation function comprises jointly optimizing the set of evaluation functions, so as to determine a set of optimized visual equipments.

In that embodiment, the set of evaluation functions are used in a multi-objective optimization process, in order to assess the problem related performance of the defined equipment: one evaluation function for each combination of object and criterion regrouping the different target points for this object and this criterion, or one evaluation function for each object regrouping the different criteria for each object, or one evaluation function for each criterion regrouping the different objects.

Aggregating several evaluation functions into one global evaluation function may be used to lower the problem complexity or, in other words, the evaluation space dimensionality.

Then, a given number of optimized equipments are determined, which are referred to as solutions, through a multi-objective optimization based on the evaluation functions previously defined. The found solution may not be unique, as optimizing various evaluation functions may lead to a group of non-dominated solutions, each expressing a different compromise. Thus, generally speaking, the optimized equipments form a Pareto front of non-dominated solutions.

A non-limiting example of multi-objective optimization is described below.

In this approach, several visual equipments, each representing various potential solutions to the multi-objective optimization problem, are tested against various performance criteria. Optimizing an equipment consists in finding the solution or set of solutions that optimize the evaluation function(s).

In this example, the optimization problem consists in finding the various visual equipments that are optimal depending on the lifestyle of a wearer. In the current example, starting from a specific set of potential solutions, it will be seen that for two identical wearers, optimizing for two different lifestyles can lead to different sets of optimal solutions.

Lifestyles will be simplified here to only specify the nature and usage of an object during a near vision task: one lifestyle implying the use of a smartphone, referred to as "digital", and one lifestyle implying the use of a standard A4-sized piece of paper, referred to as "analog". The two objects, and thus the two lifestyles, will have different positions in space, defined by a specific gaze lowering and wearer-object distance. In this example, the center of the smartphone is located along an axis at 38.4° of vertical declination from the horizontal axis passing through the average height of both eyes of the wearer, and at 0° of horizontal angle from the main gaze direction of the wearer when looking straight forward at far distance without lenses, and at a distance of 35 cm from the eyes of the wearer along this axis. This smartphone is then oriented in order to be orthogonal to this axis passing through its center. The size of the smartphone is defined as 7 cm in width and 15 cm in height. In this example, the center of the paper is located along an axis at 28° of vertical declination from the horizontal axis passing through the average height of both eyes of the wearer, and at 0° of horizontal angle from the main gaze direction of the wearer when looking straight forward at far distance without lenses, and at a distance of 43 cm from the eyes of the wearer along this axis. This paper is then oriented in order to be orthogonal to this axis passing through its center. The size of the paper is defined as 21 cm in width and 29.7 cm in height.

First, an initial wide set of 495 potential solutions was defined by linear combinations of 9 different lens designs adapted to the wearer prescription and wearing conditions, with same progression lengths and insets.

For each solution, two simulations were performed using the same wearer model, but with different lifestyles. The lifestyle modifies the position of the near vision object, as described above. During each simulation, 3 tasks were performed, corresponding to the gaze fixation on objects at near, intermediate and far distances. For each task and thus each fixation, two criteria were computed: the head comfort area and an effort value representing the combined postural and gaze effort. Thus, for each simulation, 6 criteria values were estimated.

The non-dominated solutions were selected on the basis of the optimization of the two criteria: the head comfort area had to be maximized and the postural and gaze effort had to be minimized.

Then, an algorithm was applied to find the non-dominated solutions, with the following results: 96 non-dominated solutions for all lifestyles, 72 unique solutions and 24 shared solutions. 75% of unique, lifestyle-dependent solutions were obtained. Filtering solutions associated with the lower bound 20% values, i.e. ignoring solutions corresponding to the 20% lower values among all solutions for any of the 6 criteria, remaining solutions were split as follows: 3 shared solutions and 28 unique solutions. 90% of unique, lifestyle-dependent solutions were obtained. Therefore, the method according to the present disclosure allowed the determination of optimum lens solutions depending on the lifestyle of the wearers.

Figure 4 illustrates the compromise that appears between the head comfort area criteria in the two lifestyles. The graph on the left shows values of the head comfort area for objects at near distance (i.e. in near vision, NV on the drawing) as a function of values of the head comfort area for objects at far distance (i.e. in far vision, FV on the drawing), in the "analog" lifestyle. The graph on the right shows values of the head comfort area for objects at near distance as a function of values of the head comfort area for objects at far distance, in the "digital" lifestyle. The example demonstrates the benefit of the method according to the present disclosure, which is to identify different optimal visual equipments based on the lifestyle of the wearers.

In this example, the optimal solution was found by a full sampling of the design evaluation space. This was possible because the dimension of the evaluation space was limited, as only 9 designs were combined, and the sampling was coarse. For larger evaluation spaces or finer sampling, a full sampling of the evaluation space is not applicable. In that case, multi-objective optimization methods are available, such as multi-objective optimization by genetic algorithms, as described for example in the article by A. Konak et al. entitled "Multi-objective optimization using genetic algorithms: A tutorial", in Reliability Engineering & System Safety, vol. 91, issue 9, 2006, pages 992-1007, ISSN 0951-8320, https://doi.org/10.1016/j.ress.2005.11.018.

Also, in this example, the design parametrization was obtained by linear combination of existing designs. Other design parametrizations exist and are known in the art, see for example G. Savio et al., "Parametric Modeling of Free-Form Surfaces for Progressive Addition Lens", Proceedings of the IMProVe 2011 International Conference on Innovative Methods in Product Design, June 15-17, 2011, pages 167-176.

Another non-limiting example, which is an example of merit function optimization, is described below.

In this approach, as in the previous example, the same two types of criteria are defined and each one is evaluated for the same 3 tasks, then obtaining 6 different evaluation criteria: head comfort area in far vision (FV), intermediate vision (IV) and near vision (NV) and postural and gaze efforts in FV, IV and NV.

Then, a target value and a weight are defined for each evaluation criterion, as listed in Table 1 below.

**Table 1**

| | Comfort area FV | Comfort area IV | Comfort area NV | Efforts FV | Efforts IV | Efforts NV |
|---|---|---|---|---|---|---|
| Target value | Tcafv = 1000 | Tcaiv = 50 | Tcanv = 200 | Tefv = 0.05 | Teiv = 0.15 | Tenv = 0.1 |
| Weight | Wcafv = 1 | Wcaiv = 1 | Wcanv = 1 | Wefv = 1 | Weiv = 1 | Wenv = 1 |

The following evaluation function EF(equipment) was defined in order to evaluate the performance of a visual equipment: 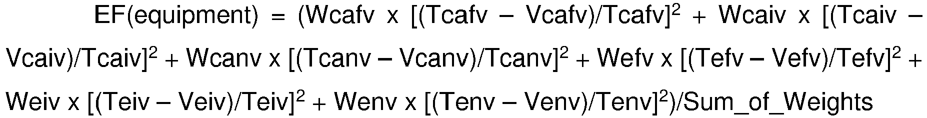 EF(equipment) = (Wcafv x [(Tcafv - Vcafv)/Tcafv]2 + Wcaiv x [(Tcaiv - Vcaiv)/Tcaiv]2 + Wcanv x [(Tcanv - Vcanv)/Tcanv]2 + Wefv x [(Tefv - Vefv)/Tefv]2 + Weiv x [(Teiv - Veiv)/Teiv]2 + Wenv x [(Tenv - Venv)/Tenv]2)/Sum_of_Weights where Vi is the value of each evaluation criterion calculated for the equipment and Sum_of_Weights is the sum of all the weights.

Considering a wearer with a prescription Plano Add +2.5 and the wearing conditions (pantoscopic tilt = -8°, wrap angle = 6.9° and cornea lens distance = 12 mm), 5 different equipments were calculated from the same product, with 5 different progression lengths (PL) from 14 mm to 18 mm with a step of 1 mm, these equipments being named E"PL" in Table 2 below. The 6 evaluation criteria were calculated for these 5 equipments for both configurations of the environment corresponding to the "analog" (A) and "digital" (D) lifestyles defined in the previous example:

**Table 2**

| | Comfort area FV | Comfort area IV | Comfort area NV | | Efforts FV | Efforts IV | Efforts NV | | Evaluation function | |
|---|---|---|---|---|---|---|---|---|---|---|
| | A/D | A/D | A | D | A/D | A/D | A | D | A | D |
| E18 | 849 | 31 | 132 | 43 | 0.08963 | 0.19836 | 0.12079 | 0.21631 | 1.0582 | 2.8684 |
| E17 | 734 | 28 | 131 | 47 | 0.08728 | 0.19458 | 0.13204 | 0.21062 | 1.0871 | 2.6743 |
| E16 | 802 | 25 | 142 | 55 | 0.08881 | 0.20064 | 0.12786 | 0.20079 | 1.1674 | 2.5472 |
| E15 | 772 | 22 | 164 | 55 | 0.08537 | 0.2067 | 0.13601 | 0.19905 | 1.1710 | 2.5156 |
| E14 | 755 | 21 | 157 | 65 | 0.08529 | 0.21533 | 0.14256 | 0.19096 | 1.3116 | 2.3673 |

For each lifestyle, the most relevant visual equipment can be determined by selecting the one corresponding to the smallest value of the evaluation function. Thus, in this example, for the "analog" lifestyle, the selected equipment is E18, corresponding to a progression length of 18 mm and for the "digital" lifestyle, the selected equipment is E14, corresponding to a progression length of 18 mm. This illustrates one of the benefits of the method according to the present disclosure, which is to be able to determine the most appropriate visual equipment taking into account the lifestyle of the wearer.

In the above example, for simplicity, a single parameter optimization was considered: only the progression length of the design was made to vary. However, the same process can be easily generalized to multiple parameters and the entire lens geometry can be made variable. In the case of a large number of parameters, the optimal solution can be found by known optimization techniques.

A device according to the invention, for determining at least one optimized visual equipment to be worn by a human wearer, comprises at least one input adapted to obtain the wearer model and to obtain the model of the at least one environment as described above.

The device also comprises at least one processor.

According to the invention, the processor is configured for determining the at least one evaluation function as described above and for optimizing the at least one evaluation function as described above, so as to determine the at least one optimized visual equipment.

The method of the invention is computer-implemented. Namely, a computer program product comprises one or more sequences of instructions that are accessible to a processor and that, when executed by the processor, cause the processor to carry out steps of the method for determining at least one optimized visual equipment as described above.

The software may be hosted for example remotely in a cloud, or locally in a computer.

The sequence(s) of instructions may be stored in one or several non-transitory computer-readable storage medium/media, including a predetermined location in a cloud.

Although representative methods and devices have been described in detail herein, those skilled in the art will recognize that various substitutions and modifications may be made without departing from the scope of what is described and defined by the appended claims.

## Claims

1. A computer-implemented method for determining at least one optimized visual equipment to be worn by a human wearer, wherein said method comprises steps, performed by a processor, of:
obtaining (10) a wearer model as a virtual model of said human wearer;
obtaining (12) a model of at least one environment for which said at least one optimized visual equipment is to be determined, said at least one environment comprising tridimensional positions of objects to be viewed by said wearer model;
determining (14) at least one evaluation function related to said visual equipment, as a function of at least optical performance of said visual equipment and postural performance of said wearer model in said model of said at least one environment, said optical performance comprising visual acuity or visual acuity loss and said postural performance comprising at least one of a head comfort area, a postural effort, a gaze effort and a combined postural and gaze effort;
optimizing (16) said at least one evaluation function, so as to determine said at least one optimized visual equipment, **characterized in that** the visual equipment is parameterized and the parameters that define said visual equipment being the variables to optimize.

2. A method according to claim 1, wherein said wearer model comprises a head (22) movable with respect to a trunk (20) and at least one eye (24) rotationally movable with respect to the head (22).

3. A method according to claim 1 or 2, wherein said at least one evaluation function comprises at least one weighting coefficient related to the human wearer's lifestyle, which relates to at least one type of activity in the course of which said human wearer intends to use said visual equipment.

4. A method according to claim 3, wherein said at least one weighting coefficient is modified for taking into account preferences or usages of said human wearer.

5. A method according to any of the preceding claims, wherein said wearer model takes into account at least one personalized parameter related to said human wearer.

6. A method according to any of the preceding claims, wherein said model of said at least one environment takes into account at least one personalized parameter related to a real environment in which said human wearer intends to use said visual equipment.

7. A method according to any of the preceding claims, wherein said method further comprises defining target optimal values related to said optical performance and said postural performance and said at least one evaluation function is a sum of weighted normalized differences between evaluated values of said optical and postural performances and said target optimal values.

8. A method according to any of the preceding claims, in which said determining (14) at least one evaluation function comprises determining a set of evaluations functions, wherein said optimizing comprises jointly optimizing said set of evaluation functions, so as to determine a set of optimized visual equipments.

9. A method according to any of the preceding claims, wherein said method further comprises defining at least one visual task by associating with each other a plurality of fixation points to be looked at by said wearer model in said model of said at least one environment and said at least one evaluation function measures said optical performance of said visual equipment and said postural performance of said wearer model when carrying out said at least one visual task.

10. A method according to claim 9, wherein said method further comprises associating a predetermined minimum visual acuity to each fixation point of said plurality of fixation points.

11. A device for determining at least one optimized visual equipment to be worn by a human wearer, wherein said device comprises:
at least one input adapted to:
obtain (10) a wearer model as a virtual model of said human wearer;
obtain (12) a model of at least one environment for which said at least one optimized visual equipment is to be determined, said at least one environment comprising tridimensional positions of objects to be viewed by said wearer model;
said device further comprises at least one processor configured for:
determining (14) at least one evaluation function related to said visual equipment, as a function of at least optical performance of said visual equipment and postural performance of said wearer model in said model of said at least one environment, said optical performance comprising visual acuity or visual acuity loss and said postural performance comprising at least one of a head comfort area, a postural effort, a gaze effort and a combined postural and gaze effort;
optimizing (16) said at least one evaluation function, so as to determine said at least one optimized visual equipment, **characterized in that** the visual equipment is parameterized and the parameters that define said visual equipment being the variables to optimize.

12. A computer program product for determining at least one optimized visual equipment to be worn by a human wearer, wherein said computer program product comprises one or more sequences of instructions that are accessible to a processor and that, when executed by said processor, cause said processor to:
obtain (10) a wearer model as a virtual model of said human wearer;
obtain (12) a model of at least one environment for which said at least one optimized visual equipment is to be determined, said at least one environment comprising tridimensional positions of objects to be viewed by said wearer model;
said processor is further configured to:
determine (14) at least one evaluation function related to said visual equipment, as a function of at least optical performance of said visual equipment and postural performance of said wearer model in said model of said at least one environment, said optical performance comprising visual acuity or visual acuity loss and said postural performance comprising at least one of a head comfort area, a postural effort, a gaze effort and a combined postural and gaze effort;
optimize (16) said at least one evaluation function, so as to determine said at least one optimized visual equipment, **characterized in that** the visual equipment is parameterized and the parameters that define said visual equipment being the variables to optimize.

13. A non-transitory information storage medium, wherein said storage medium stores one or more sequences of instructions for determining at least one optimized visual equipment to be worn by a human wearer, that are accessible to a processor and that, when executed by said processor, cause said processor to:
obtain (10) a wearer model as a virtual model of said human wearer;
obtain (12) a model of at least one environment for which said at least one optimized visual equipment is to be determined, said at least one environment comprising tridimensional positions of objects to be viewed by said wearer model;
said processor is further configured to:
determine (14) at least one evaluation function related to said visual equipment, as a function of at least optical performance of said visual equipment and postural performance of said wearer model in said model of said at least one environment, said optical performance comprising visual acuity or visual acuity loss and said postural performance comprising at least one of a head comfort area, a postural effort, a gaze effort and a combined postural and gaze effort;
optimize (16) said at least one evaluation function, so as to determine said at least one optimized visual equipment, **characterized in that** the visual equipment is parameterized and the parameters that define said visual equipment being the variables to optimize.

## Patentansprüche

1. Computerimplementiertes Verfahren zum Ermitteln mindestens einer optimierten visuellen Ausrüstung, die von einem menschlichen Träger getragen wird, wobei das Verfahren Schritte umfasst, die von einem Prozessor durchgeführt werden, zum:
Erhalten (10) eines Trägermodells als ein virtuelles Modell des menschlichen Trägers;
Erhalten (12) eines Modells von mindestens einer Umgebung, für welche die mindestens eine optimierte visuelle Ausrüstung ermittelt wird, wobei die mindestens eine Umgebung dreidimensionale Positionen von Objekten umfasst, die durch das Trägermodell betrachtet werden;
Ermitteln (14) von mindestens einer Bewertungsfunktion, die sich auf die visuelle Ausrüstung bezieht, als eine Funktion von mindestens einer optischen Leistungsfähigkeit der visuellen Ausrüstung und einer posturalen Leistungsfähigkeit des Trägermodells in dem Modell der mindestens einen Umgebung, wobei die optische Leistungsfähigkeit eine Sehschärfe oder einen Sehschärfenverlust umfasst und die posturale Leistungsfähigkeit mindestens eines von einer Kopfkomfortzone, einer posturalen Anstrengung, einer visuellen Anstrengung und einer kombinierten posturalen und visuellen Anstrengung umfasst;
Optimieren (16) der mindestens einen Bewertungsfunktion, um die mindestens eine optimierte visuelle Ausrüstung zu ermitteln, **dadurch gekennzeichnet, dass** die visuelle Ausrüstung parametriert wird und die Parameter, welche die visuelle Ausrüstung definieren, die zu optimierenden Variablen sind.

2. Verfahren nach Anspruch 1, wobei das Trägermodell einen Kopf (22), der in Bezug auf einen Rumpf (20) beweglich ist, und mindestens ein Auge (24) umfasst, das in Bezug auf den Kopf (22) drehbeweglich ist.

3. Verfahren nach Anspruch 1 oder 2, wobei die mindestens eine Bewertungsfunktion mindestens einen Gewichtungskoeffizienten umfasst, der sich auf den Lebensstil des menschlichen Trägers bezieht, der sich auf mindestens einen Aktivitätstyp bezieht, in dessen Verlauf der menschliche Träger beabsichtigt, die visuelle Ausrüstung zu verwenden.

4. Verfahren nach Anspruch 3, wobei der mindestens eine Gewichtungskoeffizient modifiziert wird, um Präferenzen von dem, oder Verwendungen durch den menschlichen Träger zu berücksichtigen.

5. Verfahren nach einem der vorhergehenden Ansprüche, wobei das Trägermodell mindestens einen personalisierten Parameter berücksichtigt, der sich auf den menschlichen Träger bezieht.

6. Verfahren nach einem der vorhergehenden Ansprüche, wobei das Modell der mindestens einen Umgebung mindestens einen personalisierten Parameter berücksichtigt, der sich auf eine reale Umgebung bezieht, in welcher der menschliche Träger beabsichtigt, die visuelle Ausrüstung zu verwenden.

7. Verfahren nach einem der vorhergehenden Ansprüche, wobei das Verfahren ferner ein Definieren von optimalen Zielwerten in Bezug auf die optische Leistungsfähigkeit und die posturale Leistungsfähigkeit umfasst und wobei die mindestens eine Bewertungsfunktion eine Summe von gewichteten normalisierten Differenzen zwischen bewerteten Werten der optischen und posturalen Leistungsfähigkeiten und den optimalen Zielwerten ist.

8. Verfahren nach einem der vorhergehenden Ansprüche, wobei das Ermitteln (14) von mindestens einer Bewertungsfunktion ein Ermitteln eines Satzes von Bewertungsfunktionen umfasst, wobei das Optimieren ein gemeinsames Optimieren des Satzes von Bewertungsfunktionen umfasst, um einen Satz von optimierten visuellen Ausrüstungen zu ermitteln.

9. Verfahren nach einem der vorhergehenden Ansprüche, wobei das Verfahren ferner ein Definieren mindestens einer visuellen Aufgabe umfasst, indem eine Vielzahl von Fixierpunkten, die von dem Trägermodell in dem Modell der mindestens einen Umgebung gesucht werden, miteinander assoziiert werden, und wobei die mindestens eine Bewertungsfunktion die optische Leistungsfähigkeit der visuellen Ausrüstung und die posturale Leistungsfähigkeit des Trägermodells misst, wenn die mindestens eine visuelle Aufgabe ausgeführt wird.

10. Verfahren nach Anspruch 9, wobei das Verfahren ferner ein Assoziieren einer vorbestimmten minimalen Sehschärfe mit jedem Fixierpunkt aus der Vielzahl von Fixierpunkten umfasst.

11. Vorrichtung zum Ermitteln mindestens einer optimierten visuellen Ausrüstung, die von einem menschlichen Träger getragen wird, wobei die Vorrichtung umfasst:
mindestens einen Eingang, der geeignet ist zum:
Erhalten (10) eines Trägermodells als ein virtuelles Modell des menschlichen Trägers;
Erhalten (12) eines Modells von mindestens einer Umgebung, für welche die mindestens eine optimierte visuelle Ausrüstung ermittelt wird, wobei die mindestens eine Umgebung dreidimensionale Positionen von Objekten umfasst, die durch das Trägermodell betrachtet werden;
wobei die Vorrichtung ferner mindestens einen Prozessor umfasst, der konfiguriert ist zum:
Ermitteln (14) von mindestens einer Bewertungsfunktion, die sich auf die visuelle Ausrüstung bezieht, als eine Funktion von mindestens einer optischen Leistungsfähigkeit der visuellen Ausrüstung und einer posturalen Leistungsfähigkeit des Trägermodells in dem Modell der mindestens einen Umgebung, wobei die optische Leistungsfähigkeit eine Sehschärfe oder einen Sehschärfenverlust umfasst und die posturale Leistungsfähigkeit mindestens eines von einer Kopfkomfortzone, einer posturalen Anstrengung, einer visuellen Anstrengung und einer kombinierten posturalen und visuellen Anstrengung umfasst;
Optimieren (16) der mindestens einen Bewertungsfunktion, um die mindestens eine optimierte visuelle Ausrüstung zu ermitteln, **dadurch gekennzeichnet, dass** die visuelle Ausrüstung parametriert wird und die Parameter, welche die visuelle Ausrüstung definieren, die zu optimierenden Variablen sind.

12. Computerprogrammprodukt zum Ermitteln mindestens einer optimierten visuellen Ausrüstung, die von einem menschlichen Träger getragen wird, wobei das Computerprogrammprodukt eine oder mehrere Anweisungssequenzen umfasst, die für einen Prozessor zugänglich sind und die, wenn sie von dem Prozessor ausgeführt werden, den Prozessor veranlassen zum:
Erhalten (10) eines Trägermodells als ein virtuelles Modell des menschlichen Trägers;
Erhalten (12) eines Modells von mindestens einer Umgebung, für welche die mindestens eine optimierte visuelle Ausrüstung ermittelt wird, wobei die mindestens eine Umgebung dreidimensionale Positionen von Objekten umfasst, die durch das Trägermodell betrachtet werden;
wobei der Prozessor ferner konfiguriert ist zum:
Ermitteln (14) von mindestens einer Bewertungsfunktion, die sich auf die visuelle Ausrüstung bezieht, als eine Funktion von mindestens einer optischen Leistungsfähigkeit der visuellen Ausrüstung und einer posturalen Leistungsfähigkeit des Trägermodells in dem Modell der mindestens einen Umgebung, wobei die optische Leistungsfähigkeit eine Sehschärfe oder einen Sehschärfenverlust umfasst und die posturale Leistungsfähigkeit mindestens eines von einer Kopfkomfortzone, einer posturalen Anstrengung, einer visuellen Anstrengung und einer kombinierten posturalen und visuellen Anstrengung umfasst;
Optimieren (16) der mindestens einen Bewertungsfunktion, um die mindestens eine optimierte visuelle Ausrüstung zu ermitteln, **dadurch gekennzeichnet, dass** die visuelle Ausrüstung parametriert wird und die Parameter, welche die visuelle Ausrüstung definieren, die zu optimierenden Variablen sind.

13. Nicht-flüchtiges Informationsspeichermedium, wobei in dem Speichermedium eine oder mehrere Anweisungssequenzen zum Ermitteln mindestens einer optimierten visuellen Ausrüstung gespeichert sind, die von einem menschlichen Träger getragen wird, die für einen Prozessor zugänglich sind und die, wenn sie von dem Prozessor ausgeführt werden, den Prozessor veranlassen zum:
Erhalten (10) eines Trägermodells als ein virtuelles Modell des menschlichen Trägers;
Erhalten (12) eines Modells von mindestens einer Umgebung, für welche die mindestens eine optimierte visuelle Ausrüstung ermittelt wird, wobei die mindestens eine Umgebung dreidimensionale Positionen von Objekten umfasst, die durch das Trägermodell betrachtet werden;
wobei der Prozessor ferner konfiguriert ist zum:
Ermitteln (14) von mindestens einer Bewertungsfunktion, die sich auf die visuelle Ausrüstung bezieht, als eine Funktion von mindestens einer optischen Leistungsfähigkeit der visuellen Ausrüstung und einer posturalen Leistungsfähigkeit des Trägermodells in dem Modell der mindestens einen Umgebung, wobei die optische Leistungsfähigkeit eine Sehschärfe oder einen Sehschärfenverlust umfasst und die posturale Leistungsfähigkeit mindestens eines von einer Kopfkomfortzone, einer posturalen Anstrengung, einer visuellen Anstrengung und einer kombinierten posturalen und visuellen Anstrengung umfasst;
Optimieren (16) der mindestens einen Bewertungsfunktion, um die mindestens eine optimierte visuelle Ausrüstung zu ermitteln, **dadurch gekennzeichnet, dass** die visuelle Ausrüstung parametriert wird und die Parameter, welche die visuelle Ausrüstung definieren, die zu optimierenden Variablen sind.

## Revendications

1. Procédé mis en œuvre par ordinateur pour la détermination d'au moins un équipement visuel optimisé à porter par un porteur humain, ledit procédé comprenant les étapes, réalisées par un processeur, de :
obtention (10) d'un modèle de porteur en tant que modèle virtuel dudit porteur humain ;
obtention (12) d'un modèle d'au moins un environnement pour lequel ledit au moins un équipement visuel optimisé est à déterminer, ledit au moins un environnement comprenant des positions tridimensionnelles d'objets à visualiser par ledit modèle de porteur ;
détermination (14) d'au moins une fonction d'évaluation liée audit équipement visuel, en fonction d'au moins une performance optique dudit équipement visuel et d'une performance posturale dudit modèle de porteur dans ledit modèle dudit au moins un environnement, ladite performance optique comprenant une acuité visuelle ou une perte d'acuité visuelle et ladite performance posturale comprenant au moins une parmi une zone de confort de tête, un effort postural, un effort de regard et un effort postural et de regard combinés ;
optimisation (16) de ladite au moins une fonction d'évaluation, de manière à déterminer ledit au moins un équipement visuel optimisé, **caractérisée en ce que** l'équipement visuel est paramétré et les paramètres qui définissent ledit équipement visuel étant les variables à optimiser.

2. Procédé selon la revendication 1, ledit modèle de porteur comprenant une tête (22) mobile par rapport à un tronc (20) et au moins un œil (24) mobile en rotation par rapport à la tête (22).

3. Procédé selon la revendication 1 ou 2, ladite au moins une fonction d'évaluation comprenant au moins un coefficient de pondération lié au mode de vie du porteur humain, qui concerne au moins un type d'activité au cours de laquelle ledit porteur humain a l'intention d'utiliser ledit équipement visuel.

4. Procédé selon la revendication 3, ledit au moins un coefficient de pondération étant modifié pour prendre en compte des préférences ou des usages dudit porteur humain.

5. Procédé selon l'une quelconque des revendications précédentes, ledit modèle de porteur prenant en compte au moins un paramètre personnalisé lié audit porteur humain.

6. Procédé selon l'une quelconque des revendications précédentes, ledit modèle dudit au moins un environnement prenant en compte au moins un paramètre personnalisé lié à un environnement réel dans lequel ledit porteur humain a l'intention d'utiliser ledit équipement visuel.

7. Procédé selon l'une quelconque des revendications précédentes, ledit procédé comprenant en outre la définition de valeurs optimales cibles liées à ladite performance optique et à ladite performance posturale et ladite au moins une fonction d'évaluation étant une somme de différences normalisées pondérées entre des valeurs évaluées desdites performances optique et posturale et lesdites valeurs optimales cibles.

8. Procédé selon l'une quelconque des revendications précédentes, ladite détermination (14) d'au moins une fonction d'évaluation comprenant la détermination d'un ensemble de fonctions d'évaluation, ladite optimisation comprenant l'optimisation conjointe dudit ensemble de fonctions d'évaluation, de manière à déterminer un ensemble d'équipements visuels optimisés.

9. Procédé selon l'une quelconque des revendications précédentes, ledit procédé comprenant en outre la définition d'au moins une tâche visuelle en associant les uns aux autres une pluralité de points de fixation devant être regardés par ledit modèle de porteur dans ledit modèle dudit au moins un environnement et ladite au moins une fonction d'évaluation mesurant ladite performance optique dudit équipement visuel et ladite performance posturale dudit modèle de porteur lors de l'exécution de ladite au moins une tâche visuelle.

10. Procédé selon la revendication 9, ledit procédé comprenant en outre l'association d'une acuité visuelle minimale prédéterminée à chaque point de fixation de ladite pluralité de points de fixation.

11. Dispositif de détermination d'au moins un équipement visuel optimisé à porter par un porteur humain, ledit dispositif comprenant :
au moins une entrée conçue pour :
obtenir (10) un modèle de porteur en tant que modèle virtuel dudit porteur humain ;
obtenir (12) un modèle d'au moins un environnement pour lequel ledit au moins un équipement visuel optimisé est à déterminer, ledit au moins un environnement comprenant des positions tridimensionnelles d'objets à visualiser par ledit modèle de porteur ;
ledit dispositif comprenant en outre au moins un processeur configuré pour :
déterminer (14) au moins une fonction d'évaluation liée audit équipement visuel, en fonction d'au moins une performance optique dudit équipement visuel et d'une performance posturale dudit modèle de porteur dans ledit modèle dudit au moins un environnement, ladite performance optique comprenant une acuité visuelle ou une perte d'acuité visuelle et ladite performance posturale comprenant au moins une parmi une zone de confort de tête, un effort postural, un effort de regard et un effort postural et de regard combinés ;
optimiser (16) ladite au moins une fonction d'évaluation, de manière à déterminer ledit au moins un équipement visuel optimisé, **caractérisée en ce que** l'équipement visuel est paramétré et les paramètres qui définissent ledit équipement visuel étant les variables à optimiser.

12. Produit programme informatique pour déterminer au moins un équipement visuel optimisé destiné à être porté par un porteur humain, ledit produit programme informatique comprenant une ou plusieurs séquences d'instructions accessibles à un processeur et qui, lorsque exécutées par ledit processeur, amènent ledit processeur à :
obtenir (10) un modèle de porteur en tant que modèle virtuel dudit porteur humain ;
obtenir (12) un modèle d'au moins un environnement pour lequel ledit au moins un équipement visuel optimisé est à déterminer, ledit au moins un environnement comprenant des positions tridimensionnelles d'objets à visualiser par ledit modèle de porteur ;
ledit processeur étant en outre configuré pour :
déterminer (14) au moins une fonction d'évaluation liée audit équipement visuel, en fonction d'au moins une performance optique dudit équipement visuel et d'une performance posturale dudit modèle de porteur dans ledit modèle dudit au moins un environnement, ladite performance optique comprenant une acuité visuelle ou une perte d'acuité visuelle et ladite performance posturale comprenant au moins une parmi une zone de confort de tête, un effort postural, un effort de regard et un effort postural et de regard combinés ;
optimiser (16) ladite au moins une fonction d'évaluation, de manière à déterminer ledit au moins un équipement visuel optimisé, **caractérisée en ce que** l'équipement visuel est paramétré et les paramètres qui définissent ledit équipement visuel étant les variables à optimiser.

13. Support de stockage d'informations non transitoire, ledit support de stockage stockant une ou plusieurs séquences d'instructions pour déterminer au moins un équipement visuel optimisé devant être porté par un porteur humain, qui sont accessibles à un processeur et qui, lorsqu'elles sont exécutées par ledit processeur, amènent ledit processeur à :
obtenir (10) un modèle de porteur en tant que modèle virtuel dudit porteur humain ;
obtenir (12) un modèle d'au moins un environnement pour lequel ledit au moins un équipement visuel optimisé est à déterminer, ledit au moins un environnement comprenant des positions tridimensionnelles d'objets à visualiser par ledit modèle de porteur ;
ledit processeur étant en outre configuré pour :
déterminer (14) au moins une fonction d'évaluation liée audit équipement visuel, en fonction d'au moins une performance optique dudit équipement visuel et d'une performance posturale dudit modèle de porteur dans ledit modèle dudit au moins un environnement, ladite performance optique comprenant une acuité visuelle ou une perte d'acuité visuelle et ladite performance posturale comprenant au moins une parmi une zone de confort de tête, un effort postural, un effort de regard et un effort postural et de regard combinés ;
optimiser (16) ladite au moins une fonction d'évaluation, de manière à déterminer ledit au moins un équipement visuel optimisé, **caractérisée en ce que** l'équipement visuel est paramétré et les paramètres qui définissent ledit équipement visuel étant les variables à optimiser.
